# EUROPEAN PATENT APPLICATION

(11) **EP 4 609 860 A1**
(43) Date of publication of application: **03.09.2025**
(21) Application number: 23883119.2
(22) Date of filing: 26.10.2023
(51) Int. Cl.: A61K 31/19, A61P 27/16, A23L 33/10

(54) **PHARMACEUTICAL COMPOSITION FOR PREVENTING OR TREATING HEARING LOSS COMPRISING AMMONIUM LACTATE AS ACTIVE INGREDIENT**

(30) Priority: 27.10.2022 KR 20220140306
(71) Applicant: Korea University Research and Business Foundation, Seoul 02841 (KR)
(72) Inventor: CHOI, June, Ansan-si Gyeonggi-do 15483 (KR); LEE, Sang Hyun, Seoul 02616 (KR); HAN, Eunjung, Daegu 42606 (KR); PARK, Saemi, Ansan-si Gyeonggi-do 15467 (KR); JO, Yuji, Ansan-si Gyeonggi-do 15401 (KR); CHOI, Yunjae, Seoul 02477 (KR)
(74) Representative: Mewburn Ellis LLP
(86) International application number: PCT/KR2023/016730
(87) International publication number: WO 2024/091014

(57) **Abstract**

The present invention relates to a pharmaceutical composition for preventing or treating hearing loss, comprising ammonium lactate as an active ingredient. Since it was confirmed that the number of normal sensory hair cells increased when sensory hair cells damaged by gentamicin was treated with ammonium lactate, a pharmaceutical composition comprising ammonium lactate as an active ingredient can be used to prevent or treat hearing loss as a side effect due to use of ammonium lactate, which is an antibiotic.

## Description

### Technical Field

The present disclosure provides a pharmaceutical composition for preventing or treating a hearing loss, including ammonium lactate as an active ingredient.

### Background Art

Gentamicin is a drug that has been widely used as an excellent antibiotic for a long time, which is approved by the United States Food and Drug Administration. It is an aminoglycoside-based antibiotic substance produced by Micromonospora purpurea and Micromonospora echinospora, and is a mixture of gentamicin C1, C2, and C1a. It is effective against gram-positive bacteria such as Staphylococcus aureus and gram-negative bacteria such as Escherichia coli, Salmonella, Proteus, and Pseudomonas aeruginosa. Gentamicin is also used in the treatment of enterococcal endocarditis in combination with penicillin or ampicillin. It is often administered parenterally or used as an external medication.

A recent study announced that gentamicin is able to completely block progression of Duchenne muscular dystrophy on which antibiotic administration takes effect. However, permanent hearing loss and kidney damage are known to be representative side effects.

Ototoxic hearing loss is a side effect caused by the use of ototoxic drugs, and aminoglycoside antibiotics and some anticancer drugs are known to cause ototoxicity. Aminoglycoside antibiotics exhibit an antibacterial action by binding to bacterial 30S ribosomal subunits to inhibit protein synthesis, such that when these aminoglycoside antibiotics are continuously exposed to cochlear cells, it results in apoptosis of outer hair cells and sensory hair cells in the organ of Corti. The mechanism of apoptosis is known to be caused by reactive oxygen species (ROS) formed by aminoglycoside antibiotics and Ca++ complexes.

A majority of protective drugs for ototoxic hearing loss that has been developed to date and undergoing clinical trials are based on a mechanism that blocks oxygen radicals. Drugs based on the reactive oxygen radical scavenging mechanism have an efficacy to suppress production of reactive oxygen by forming complexes with antibiotics owing to strong nucleophiles, but their antibiotic effects also degrade thereby, bearing a fundamental limitation in its effectiveness that is minimal due to their non-specific mechanism.

Therefore, there is a need to develop a treatment that is effective in treating ototoxic hearing loss without interfering with the effectiveness of antibiotics.

### Disclosure of the Invention

### Technical Goals

An object of the present disclosure is to provide a pharmaceutical composition for preventing or treating a hearing loss, including ammonium lactate as an active ingredient.

Another object of the present disclosure is to provide a health functional food composition for preventing or ameliorating a hearing loss, including ammonium lactate as an active ingredient.

### Technical Solutions

To achieve the above object, the present disclosure provides a pharmaceutical composition for preventing or treating a hearing loss, including ammonium lactate as an active ingredient.

In addition, the present disclosure provides a health functional food composition for preventing or ameliorating a hearing loss, including ammonium lactate as an active ingredient.

### Advantageous Effects

The present disclosure relates to a pharmaceutical composition for preventing or treating a hearing loss including ammonium lactate as an active ingredient, wherein it was found that the number of normal hair cells increases by treating ammonium lactate on hair cells damaged by gentamicin, such that the pharmaceutical composition including ammonium lactate as an active ingredient may be utilized for preventing or treating a hearing loss, which is a side effect caused by use of ammonium lactate, an antibiotic.

### Brief Description of Drawings

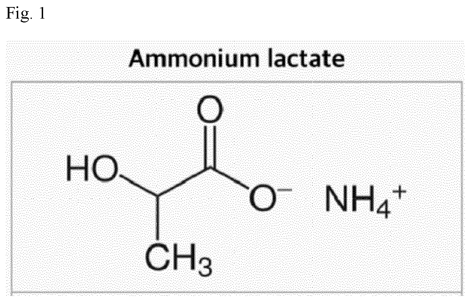

FIG. 1 shows a molecular structure of ammonium lactate.
FIG. 2 shows a result of determining the number of hair cells in zebrafish following administration of ammonium lactate.
FIG. 3 shows a result of identifying a viability of hair cells in zebrafish following administration of ammonium lactate.

### Best Mode for Carrying Out the Invention

Hereinafter, the present disclosure will be described in more detail.

The present disclosure provides a pharmaceutical composition for preventing or treating a hearing loss, including ammonium lactate as an active ingredient.

The hearing loss may be one or more from the group consisting of an ototoxic hearing loss, a sudden hearing loss, a noise-induced hearing loss, and an age-related hearing loss.

The ototoxic hearing loss may be a hearing loss caused by an anticancer drug or an aminoglycoside-based antibiotic.

The anticancer drug may be cisplatin or carboplatin.

The aminoglycoside-based antibiotic may be selected from the group consisting of amikacin, arbekacin, kanamycin, gentamicin, neomycin, netilmicin, dibekacin, sisomycin, streptomycin, tobramycin, livodomycin, and paromomycin.

In other embodiments of the present disclosure, the pharmaceutical composition may further include one or more additives selected from the group consisting of appropriate carriers, excipients, disintegrators, sweeteners, coating agents, leavening agents, lubricants, glidants, flavoring agents, antioxidants, buffers, bacteriostatic agents, diluents, dispersants, surfactants, binders, and lubricants that are commonly used in the manufacture of pharmaceutical compositions. Specifically, lactose, dextrose, sucrose, sorbitol, mannitol, xylitol, erythritol, maltitol, starch, gum acacia, alginate, gelatin, calcium phosphate, calcium silicate, cellulose, methylcellulose, microcrystalline cellulose, polyvinyl pyrrolidone, water, methyl hydroxybenzoate, propyl hydroxybenzoate, talc, magnesium stearate, and mineral oil may be used as the carriers, excipients, and diluents, solid preparations for oral administration may include tablets, pills, powders, granules, and capsules, and these solid preparations may be prepared by mixing, in the composition, at least one or more excipients, for example, starch, calcium carbonate, sucrose or lactose, and gelatin. In addition to simple excipients, lubricants such as magnesium stearate and talc may also be used. Oral liquid preparations may include suspensions, liquids, emulsions, and syrups, and various excipients, such as humectants, sweeteners, fragrances, and preservatives may be included in addition to the simple diluents that are commonly used, such as water and liquid paraffin. Preparations for parenteral administration include sterile aqueous solutions, non-aqueous solvents, suspensions, emulsions, lyophilized agents, and suppositories. For non-aqueous solvents and suspensions, propylene glycol, polyethylene glycol, vegetable oils such as olive oil, and injectable esters such as ethyl oleate may be used. As for base materials of suppositories, witepsol, macrogol, tween 61, cacao butter, laurin fat, and glycerogelatin may be used. According to one embodiment of the present disclosure, the pharmaceutical composition may be administered to a subject in a conventional manner through intravenous, intraarterial, intraperitoneal, intramuscular, intraarterial, intraperitoneal, intrasternal, transdermal, intranasal, inhalation, topical, rectal, oral, intraocular, or intradermal routes. The dosage of the active ingredient according to the present disclosure may vary depending on the condition and body weight of the subject, the type and severity of the disease, the form of the drug, and the route and duration of administration and may be appropriately selected by those skilled in the art, and the daily dose may be 0.01 mg/kg to 200 mg/kg, preferably 0.1 mg/kg to 200 mg/kg, and more preferably 0.1 mg/kg to 100 mg/kg. The administration may be conducted once a day or divided into several doses, but the scope of the present disclosure is not limited thereby.

In addition, the present disclosure provides a health functional food composition for preventing or ameliorating a hearing loss, including ammonium lactate as an active ingredient.

The health functional food may contain various nutritional supplements, vitamins, minerals (electrolytes), flavor agents such as synthetic flavor agents and natural flavor agents, coloring agents and thickening agents (cheese, chocolate, etc.), pectic acid and salts thereof, alginate and salts thereof, organic acids, protective colloidal thickeners, pH regulators, stabilizers, preservatives, glycerin, alcohol, and carbonating agents used in carbonated beverages. In addition, it may contain pulp for the manufacture of natural fruit juices, synthetic fruit juices, and vegetable drinks. These ingredients may be used independently or in combination. In addition, the health functional food composition may be in any one form of meat, sausage, bread, chocolate, candy, snacks, confectionery, pizza, instant noodles, chewing gum, ice cream, soups, beverages, teas, functional waters, drinks, alcohol, and vitamin complexes. In addition, the health functional food may further include food additives, and the suitability as the "food additive" is determined by the standards and criteria related to corresponding items according to the general rules and general test methods of Korean Food Additives Codex approved by the Ministry of Food and Drug Safety, unless otherwise stipulated. The items listed in the "Korean Food Additives Codex" may include, for example, chemically synthesized compounds such as ketones, glycine, potassium citrate, nicotinic acid, and cinnamic acid, natural additives such as persimmon color, licorice extracts, crystallized cellulose, kaoliang color, and guar gum, and mixed preparations such as sodium L-glutamate preparations, noodle-added alkali agents, preservative agents, and tar color agents. Here, the active ingredient added to the food in the process of manufacturing the health functional food may be appropriately adjusted as needed, and preferably it may be added to be included in an amount of 1 part by weight to 90 parts by weight per 100 parts by weight of food.

Hereinafter, the present disclosure will be described in more detail through examples to help understanding of the present disclosure. However, examples below are merely intended to illustrate the present disclosure, and the scope of the present disclosure is not limited to the following examples. Examples of the present disclosure are provided to more completely explain the present disclosure to those skilled in the art.

### Modes for Carrying Out the Invention

### <Experimental Example 1> Breeding of zebrafish

Zebrafish larvae (Wild type, Brn3c:EGFP, etc.) were cultured in the breeding room of our research institute. Each larva was cultured in embryo medium (15 mM NaCl, 0.5 mM KCl, 1mM CaCl₂, 1 mM MgSO₄, 0.15 mM KH₂PO₄, 0.05 mM NH₂PO₄, and 0.7 mM NaHCO₃).

### <Experimental Example 2> Analysis of hair cells in zebrafish

Gentamicin, an ototoxicity-inducing substance, was administered to zebrafish 5 days after fertilization, and the concentration of cisplatin (LC 50) that induces 50% damage to hair cells in the lateral line of zebrafish as well as the time were determined. In the study results, 50 µM gentamicin was chosen since approximately 50% of the hair cells were damaged when administered for 1 hour. The effect was observed when 10 µM, 100 µM, 500 µM, and 1000 µM ammonium lactate were co-administered with 50 µM gentamicin for 1 hour.

There were four sites in hair cells of zebrafish, including orbits (SO1 and SO2), ear (O1), and larynx (OC1), and the number of hair cells was analyzed using a microscope (fluorescence microscope, confocal microscope, etc.). The effects of candidate substances capable of suppressing damage to hair cells caused by gentamicin were compared and analyzed by administering the candidate substances to zebrafish larvae at different concentrations.

### <Example 1> Verification of protective effects of ammonium lactate on hair cells in zebrafish

To verify the protective effect of ammonium lactate on hair cells in zebrafish, ammonium lactate and gentamicin were co-administered to zebrafish, and the viability of hair cells was compared with that of the control group.

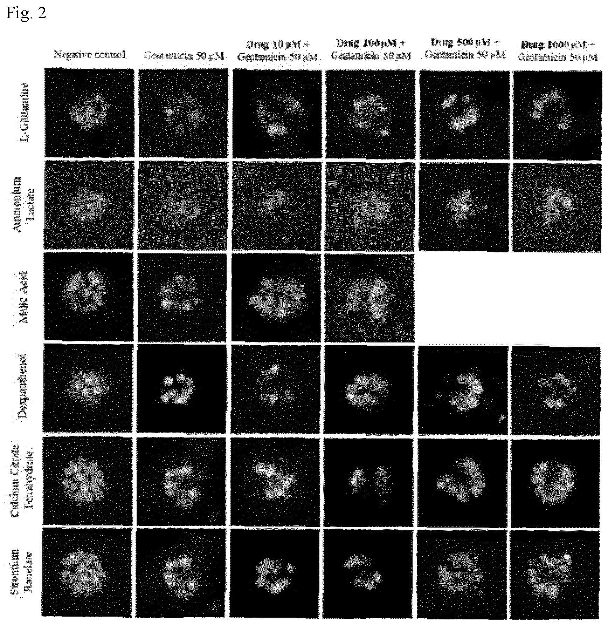

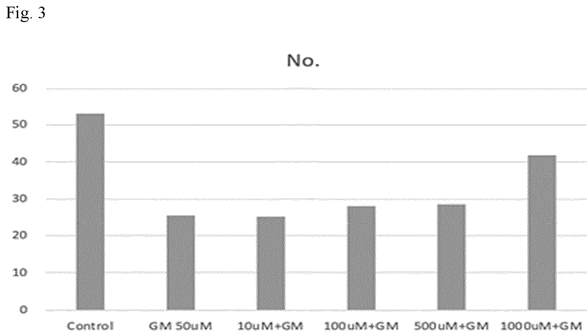

As a result, according to FIGS. 2 and 3, it was found that the number of hair cells in the control group with no treatment was 53, and in the control group administered only with 50 µM gentamicin with no administration of ammonium lactate, the number of hair cells was 26, showing a 50% decrease in the viability, after which the viability of hair cells increased in a concentration-dependent manner in the group co-administrated with 10 µM, 100 µM, 500 µM, and 1000 µM ammonium lactate along with 50 µM gentamicin. In particular, in the case of 100 µM and 500 µM ammonium lactate, the number of hair cells increased to 28 and 29, with approximately 10% increase in the viability, while in the case of 1000 µM ammonium lactate, the number of hair cells was 42 in total, showing a 60% or greater increase in the viability.

Therefore, it was found that ammonium lactate has a protective effect on hair cells.

The foregoing description of the present disclosure is for illustrative purposes only, and those skilled in the art to which the present disclosure pertains will appreciate that the present disclosure may be easily modified into other specific forms without changing the technical idea or essential characteristics of the present disclosure. Therefore, it should be understood that the embodiments describe above are exemplary in all respects and not limiting.

The scope of the present disclosure is indicated by the appended claims, and all changes or modifications derived from the meaning and scope of the claims and equivalent concepts thereof should be construed as being included in the scope of the present disclosure.

## Claims

1. A pharmaceutical composition for preventing or treating a hearing loss, comprising ammonium lactate as an active ingredient.

2. The pharmaceutical composition of claim 1, wherein the hearing loss is one or more from the group consisting of an ototoxic hearing loss, a sudden hearing loss, a noise-induced hearing loss, and an age-related hearing loss.

3. The pharmaceutical composition of claim 2, wherein the ototoxic hearing loss is a hearing loss caused by an anticancer drug or an aminoglycoside-based antibiotic.

4. The pharmaceutical composition of claim 3, wherein the anticancer drug is cisplatin or carboplatin.

5. The pharmaceutical composition of claim 3, wherein the aminoglycoside-based antibiotic is one or more selected from the group consisting of amikacin, arbekacin, kanamycin, gentamicin, neomycin, netilmicin, dibekacin, sisomycin, streptomycin, tobramycin, livodomycin, and paromomycin.

6. A health functional food composition for preventing or ameliorating a hearing loss, comprising ammonium lactate as an active ingredient.
